# EUROPÄISCHE PATENTANMELDUNG

(11) **EP 1 952 840 A1**
(43) Veröffentlichungstag der Anmeldung: **06.08.2008**
(21) Anmeldenummer: 08008087.2
(22) Anmeldetag: 01.04.2005
(51) Int. Cl.: A61N 1/375, H01R 13/18, H01R 13/187

(54) **Kontaktanordnung**

(30) Priorität: 05.04.2004 DE 102004017659
(62) Teilanmeldung aus: 05102602.9
(71) Anmelder: BIOTRONIK CRM Patent AG, 6341 Baar (CH)
(72) Erfinder: Olbertz, Ralf, 12347 Berlin (DE)
(74) Vertreter: Lindner-Vogt, Karin L.

(57) **Zusammenfassung**

Die Erfindung betrifft eine Kontakt-Anordnung zum Anschluss einer Elektrodenleitung an ein implantierbares Gerät, mit einer elektrischen Anschlussbuchse mit einer Buchsenlängsachse und wenigstens einer Öffnung zum Einführen eines Steckers entlang der Buchsenlängsachse. Die Kontakt-Anordnung umfasst mindestens ein elektrisch leitfähiges Federkontaktelement welches ausgebildet und angeordnet ist, beim Einführen eines Steckers federnd elastisch nach außen auszuweichen dabei in einer quer zur Buchsenlängsachse verlaufenden Federauslenkungsrichtung komprimiert zu werden und eine dem Komprimieren entgegenwirkende Federkraft auszubilden. Die Kontakt-Anordnung ist dadurch gekennzeichnet, dass das Feder-Kontaktelement eine einstückige, metallische Federkontaktzunge mit quer zur Federauslenkungsrichtung verlaufenden Federkontaktzungenabschnitten aufweist, die durch Wendeabschnitte miteinander verbunden sind, wobei in Federauslenkungsrichtung unmittelbar aufeinander folgende Wendeabschnitte mit jeweils entgegengesetztem Wenderichtungssinn zueinander hingewendet sind.

## Beschreibung

Die Erfindung betrifft eine Kontakt-Anordnung zum Anschluss einer Elektrodenleitung an ein implantierbares Gerät. Die Kontakt-Anordnung weist eine elektrische Anschlussbuchse mit einer Buchsenlängsachse und wenigstens einer Öffnung zum Einführen eines Steckers entlang der Buchsenlängsachse auf. Die Kontakt-Anordnung weist auch mindestens ein elektrisch leitfähiges Federkontaktelement auf, welches ausgebildet und angeordnet ist, beim Einführen eines Steckers federnd elastisch nach außen auszuweichen, dabei wenigstens in einer quer zur Buchsenlängsachse verlaufenden Federauslenkungsrichtung komprimiert zu werden und eine dem Komprimieren entgegenwirkende Federkraft auszubilden.

Feder-Kontaktelemente für implantierbare Geräte sind aus dem Stand der Technik bekannt. Aus der DE 196 09 367 der Anmelderin ist ein Herzschrittmacher mit einer rund ausgebildeten Hülse zur Aufnahme einer Elektrodenleitung bekannt, welche radial nach innen federnde Feder-Kontaktelemente aufweist, welche gleichmäßig am Hülsenumfang verteilt sind. Die Feder-Kontaktelemente sind streifenförmig ausgebildet, einseitig an der Hülsenwand befestigt und weisen in den Hülsenraum, wobei ihr freies Ende halbkugelförmig konvex ausgebildet ist und mit der konvexen Seite radial nach innen weist.

Solche Feder-Kontaktelemente haben das Problem, dass sich die Kontaktfeder-Andruckkraft nur schwierig einstellen lässt, insbesondere, wenn - wie oft üblich - das Federelement aus der Hülsenwand ausgestanzt und die somit erhaltene Blattfeder in den Innenraum gebogen ist. Hinsichtlich der Materialauswahl zur Herstellung der Feder ist man hier auf das Hülsenmaterial angewiesen, so dass in einem solchen Fall stets ein Kompromiss zwischen Anforderungen an eine Hülse und den Anforderungen an eine Kontaktfeder zur Herstellung eines sicheren und guten elektrischen Wirkkontaktes mit einer Elektrodenleitung gebildet wird.

Der Erfindung liegt die Aufgabe zugrunde, ein Kontaktelement anzugeben, welches elektrische Kontakte wirksam vor eindringender Feuchtigkeit, insbesondere vor Körperflüssigkeit, schützt.

Die Aufgabe wird gelöst durch eine Kontakt-Anordnung (101, 201) für eine Elektrodenleitungs-Anschlussbuchse für ein implantierbares Gerät, wobei die Kontakt-Anordnung eine Hülse (105) mit einer Hülsenlängsachse (114, 214) und einer Hülsenwand (106, 203) umfasst, wobei die Hülsenwand (106, 203) einen Hülseninnenraum (104) umschließt, welcher wenigstens eine Öffnung zum Einführen eines Elektrodenleitungs-Steckers entlang der Hülsenlängsachse (114, 214) aufweist. Die Hülse (105) weist eine Dichtung (211) auf, welche einen runden Durchbruch (213) zum Einführen eines Elektrodenleitungs-Steckers mit rundem Querschnitt aufweist und derart geformt ist, dass die Öffnung gegen die Hülsenwand (106, 203) und gegen den Durchbruch (213) abgedichtet ist.

Die Dichtung mit einem runden Durchbruch zur Aufnahme einer Elektrodenleitung ist derart geformt ist, dass die Öffnung gegen die Hülsenwand und gegen den Durchbruch - und somit im Anwendungsfall gegen eine Elektrodenleitung - abgedichtet ist. Bevorzugt hat der Durchbruch einen kreisrunden Querschnitt. Dadurch sind Federkontaktelemente wirksam gegen eindringende Körperflüssigkeit und somit auch gegen Korrosion geschützt.

Weiter bevorzugt ist eine Dichtung eine Dichtscheibe, welche einen Durchbruch mit einer Durchbruch-Innenwand aufweist, wobei die Durchbruch-Innenwand wenigstens zwei in Hülsenlängsachse hintereinander angeordnete Dichtlippen aufweist, welche jeweils radial nach innen - und somit in Richtung Buchsenlängsachse - weisen und welche an die Dichtscheibe angeformt sind. Die Dichtlippen bewirken gemeinsam eine besonders gute Dichtwirkung, insbesondere wenn die Dichtwirkung einer Dichtlippe, beispielsweise durch Beschädigung oder Unebenheiten einer in die Anschlussbuchse eingeführten Elektrodenleitung, verloren geht.

Die Dichtscheibe ist bevorzugt derart angeordnet, dass die Hülsenlängsachse senkrecht zur Dichtscheiben-Ebene verläuft.

Weiter bevorzugt ist die Dichtscheibe an die Hülse angeformt. In einer bevorzugten Ausführungsform weist die Dichtscheibe im Bereich des äußeren Dichtscheibenrandes eine umlaufende Nut auf, welche bevorzugt die Querschnittsform der Hülsenwand aufweist.

Bevorzugt ist die Nut eine kreisförmige Ring-Nut. Der Ring-Nut-Durchmesser entspricht bevorzugt dem Hülsendurchmesser, so dass das Ende einer Hülsenwand mit offener Stirnseite in die Ring-Nut der Dichtscheibe eingeführt werden kann.

Unabhängig von einer Anordnung mit einem zuvor beschriebenen Feder-Kontaktelement kann eine Anordnung mit einer Elektrodenleitungs-Anschlussbuchse - ohne ein zuvor beschriebenes Federkontaktelement - eine zuvor beschriebene Hülse mit einer zuvor beschriebenen Dichtung aufweisen. Dadurch sind in der Anschlussbuchse befindliche elektrische Kontakte wirksam vor eindringender Feuchtigkeit geschützt.

In einer bevorzugten Ausführungsform weist eine Feder-Kontaktelement-Anordnung für eine Kontakt-Anordnung einen Feder-Kontaktelement-Träger mit mindestens drei, bevorzugt vier, weiter bevorzugt sechs angeformten Feder-Kontaktelementen auf. Der Feder-Kontaktelement-Träger ist als kreisrunde flache Scheibe ausgebildet, welche einen - bevorzugt zentrisch angeordneten - Durchbruch zur Durchführung eines Steckers aufweist.

In einer weiter bevorzugten Ausführungsform sind die Feder-Kontaktelemente am Feder-Kontaktelement-Träger in Umfangsrichtung gleichmäßig angeordnet und im Bereich des äußeren Randes des Feder-Kontaktelement-Trägers angeformt.

Besonders bevorzugt sind die Feder-Kontaktelemente jeweils aus einer wellenförmig gewendeten Blattfederzunge gebildet, wobei die Blattfederzunge zwei Wendeabschnitte mit jeweils einander entgegengesetztem Wenderichtungssinn aufweist und so eine S-förmige Wellenform hat. Die Blattfederzungen sind ausgebildet, unter Auslenkung entlang einer Federauslenkungsachse eine der Auslenkung entgegenwirkende Federkraft auszubilden und beim Einführen eines Steckers federnd elastisch nach außen auszuweichen.

In einer vorteilhaften Ausführungsvariante weist der Feder-Kontaktelement-Träger in Umfangsrichtung jeweils seitlich an die Federkontaktelemente anschließende Aussparungsbereiche aufweist.

Weiter bevorzugt weist der Feder-Kontaktelement-Träger in Umfangsrichtung an der Innenkante, welche an den Durchbruch des Feder-Kontaktelement-Trägers angrenzt, jeweils gegenüber eines jeden Federkontaktelements angeordnete Aussparungsbereiche auf. Die Aussparungen am inneren Rand des Feder-Kontaktelement-Trägers zum Durchbruch hin sind bevorzugt derart tief in radiale Richtung nach außen eingeschnitten, dass ein noch stehender Bereich des Feder-Kontaktelement-Trägers zwischen den äußeren und den inneren Aussparungen - gesehen in radialer Richtung - eine in tangentialer Richtung wirkende Torsionsfeder bildet. Die Torsionsfeder wirkt in Serie zu den Federkontaktelementen und erleichtert vorteilhaft das Einführen eines Steckers in eine Anschlussbuchse.

In einer bevorzugten Ausführungsvariante ist der Feder-Kontaktelement-Träger mit den Federkontaktelementen aus einem einstückigen Blech gebildet, welches strahlenförmig radial nach außen weisende Blattfederstreifen aufweist. Die Feder-Kontaktelemente sind jeweils aus den strahlenförmig radial nach außen weisenden Blattfederstreifen gebildet, wobei die Blattfederstreifen am Umfangsansatz des Feder-Kontaktelement-Trägers - bevorzugt rechtwinklig - abgewinkelt und im weiteren Verlauf zu einer S-förmigen Wellenform ausgeformt sind.

Der Feder-Kontaktelement-Träger mit den Federkontaktelementen kann aus einem Blech gestanzt oder lasergeschnitten sein.

In einer vorteilhaften Ausführungsform sind die Aussparungen seitlich der Federkontaktelemente derart tief in radiale Richtung eingeschnitten, dass die Aussparungen seitlich der Federkontaktelemente derart tief in radiale Richtung eingeschnitten sind, dass nach mindestens rechtwinkligem Abwinkeln eines Blattfederstreifens ein Abschnitt des Blattfederstreifens beginnend am Umfang des Feder-Kontaktelement-Trägers (215) einen äußeren Umfangsradius des Feder-Kontaktelement-Trägers (215) neben den Aussparungen nicht überschreitet.

In einer bevorzugten Ausführungsform umfasst die Anordnung auch eine Hülse mit einer Hülsenlängsachse und einer Hülsenwand. Die Hülsenwand umschließt einen Hülseninnenraum, welcher wenigstens eine Öffnung zum Einführen eines Elektroden-Steckers in Richtung der Hülsenlängsachse aufweist.

In der hier beschriebenen Ausführungsform sind die Hülsenlängsachse und die Buchsenlängsachse der Anschlussbuchse zueinander parallel oder bilden eine gemeinsame Achse. Das Feder-Kontaktelement kann wenigstens teilweise in dem Hülseninnenraum angeordnet sein.

In einer Ausführungsform weist die Hülsenwand einen Durchbruch zur Aufnahme eines Feder-Kontaktelements auf, wobei das Feder-Kontaktelement derart in dem Durchbruch angeordnet ist, dass mindestens ein zum elektrischen Kontaktieren eines Elektrodenleitungs-Steckers vorgesehener Abschnitt des Feder-Kontaktelements in dem Hülseninnenraum angeordnet ist.

In einer besonders bevorzugten Ausführungsform ist die Hülsenwand zylindrisch ausgebildet und der Hülseninnenraum weist in Richtung der Hülsenlängsachse zwei Öffnungen zur Aufnahme eines Elektroden-Steckers auf, so dass die Hülse in Richtung der Hülsenlängsachse vollständig durchbrochen ist. Die Hülsenwand kann auch einen halbkreisförmigen, rechteckigen oder quadratischen Querschnitt haben.

Weiter bevorzugt weist die Hülse mindestens drei, besonders bevorzugt sechs Feder-Kontaktelemente auf, wobei die Feder-Kontaktelemente in einer gemeinsamen, senkrecht zur Buchsenlängsachse verlaufenden Ebene angeordnet sind.

Besonders vorteilhaft ist die Kombination von einer Dichtscheibe mit einem Federkontaktelement-Träger mit nach innen ragenden Federkontaktelementen, die innerhalb einer gemeinsamen Hülse in Hülsenlängsrichtung hintereinander angeordnet sind.

Die Erfindung betrifft auch ein implantierbares Gerät mit einer zuvor beschriebenen Kontakt-Anordnung. Das implantierbare Gerät kann ein Herzschrittmacher, ein Kardioverter, ein Defibrillator oder eine Kombination aus diesen sein. Die Elektrodenleitungs-Anschlussbuchse ist bevorzugt im Header eines implantierbaren Gerätes angeordnet.

Die Erfindung soll nun im Folgenden anhand der Figuren beschrieben werden.
- Figur 1: zeigt schematisch einen Längsschnitt eines Ausführungsbeispiels einer Anordnung für eine Elektrodenleitungs-Anschlussbuchse mit einer Hülse und Feder-Kontaktelementen.
- Figur 2a: zeigt schematisch einen Längsschnitt einer Anordnung mit einer Hülse, welche Feder-Kontaktelemente und eine Dichtung aufweist.
- Figur 2b: zeigt schematisch eine Explosionsdarstellung der in Figur 2 in Längsschnitt gezeigten Anordnung.
- Figuren 3a und b: zeigen eine Alternative zur der in Figur 2 abgebildeten Ausführungsvariante.

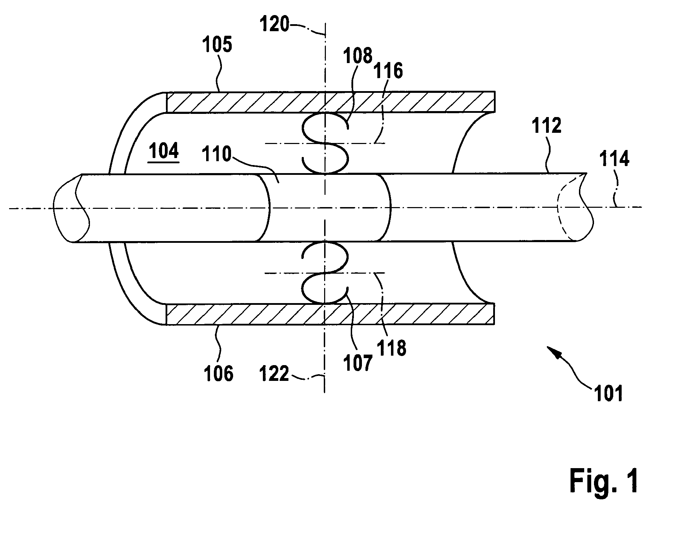

Figur 1 zeigt - schematisch dargestellt - einen Längsschnitt einer Anordnung 101 für eine Elektrodenleitungs-Anschlussbuchse eines implantierbaren Geräts.

Die Anordnung 101 umfasst eine Hülse 105 mit einer zentrisch verlaufenden Hülsenlängsachse 114, wobei die Hülse 105 einen Hülseninnenraum 104 umschließt.

Die Hülse 105 kann in einer Elektrodenleitungsbuchse derart angeordnet sein, dass die Hülsenlängsachse 114 mit einer Buchsenlängsachse eine gemeinsame Achse bildet oder zu dieser parallel ist.

Der Hülseninnenraum 104 ist an den Enden der Hülse 105 jeweils geöffnet, so dass ein - in dieser Figur abschnittsweise dargestellter - Stecker einer Elektrodenleitung 112 entlang der Hülsenlängsachse 114 durchgängig in den Hülseninnenraum 104 eingeführt werden kann.

Die Hülse 105 umfasst eine Hülsenwand 106, wobei an der Innenseite der Hülsenwand 106 Feder-Kontaktelemente 107 und 108 in radialer Richtung nach innen weisend angeordnet sind. Die Feder-Kontaktelemente 108 und 107 sind in dem Hülseninnenraum angeordnet, in radialer Richtung der Hülsenlängsachse 114 und somit auch dem in dieser Abbildung dargestellten eingeführten Stecker einer Elektrodenleitung 112 entgegenzufedern.

Das Feder-Kontaktelement 107 ist aus einer metallischen Federzunge gebildet und parallel zu einer Federquerachse 118 alternierend entgegengesetzt gewendet, so dass das Feder-Kontaktelement 107 zwei Wendeabschnitte mit jeweils entgegengesetztem Wenderichtungssinn aufweist. Das Feder-Kontaktelement 107 ist ausgebildet, unter Auslenkung wenigstens entlang einer Feder-Auslenkungsachse 122 eine der Auslenkung entgegenwirkende Kraft auszubilden. Die Auslenkung wird in diesem Ausführungsbeispiel durch den entlang der Hülsenlängsachse 114 eingeführten Stecker einer Elektrodenleitung 112 entlang der Federauslenkungsachse 122 radial nach außen, also in Richtung Hülsenwand 106 bewirkt, so dass das Feder-Kontaktelement 107 komprimiert wird.

Die Hülse 105 umfasst auch ein an der Innenseite der Hülsenwand 106 angeordnetes Feder-Kontaktelement 108.

Das Feder-Kontaktelement 108 ist genauso wie das Feder-Kontaktelement 107 aus einer metallischen Federzunge gebildet und weist auch zwei Wendeabschnitte mit jeweils entgegengesetztem Wenderichtungssinn auf.

Die Federkontaktzunge kann einen quadratischen oder runden Querschnitt aufweisen.

Vorteilhafte Ausführungsformen einer Federkontaktzunge weisen eines oder eine Kombination der folgenden Materialien auf:

Hochlegierte Stähle nach DIN 17224: X 12 CrNi 17 7; X 7 CrNiAl 17 7; oder X 5 CrNiMo 18 10 oder andere hochlegierte Metalllegierungen.

Eine Federkontaktzunge kann wenigstens abschnittweise vorteilhaft versilbert, vergoldet, mit Platinbromid (PtBr) beschichtet oder eine Kombination aus diesen Beschichtungen aufweisen. Beispielsweise ist eine Federkontaktzunge verkupfert und obenauf versilbert.

Das Feder-Kontaktelement 108 ist ausgebildet, unter Auslenkung entlang einer Federauslenkungsachse 120 eine der Auslenkung entgegenwirkende Federkraft auszubilden. Die Federauslenkungsachse 120 und die Federquerachse 116 sind zueinander orthogonal; die Federauslenkungsachse 122 und die Federquerachse 116 sind auch zueinander orthogonal.

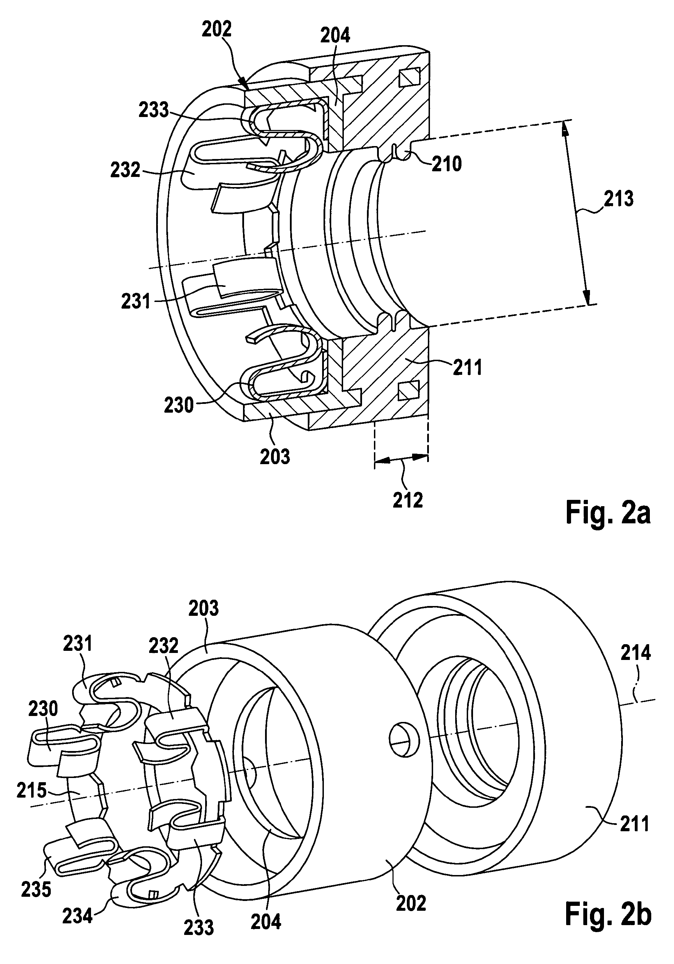

Figur 2a zeigt - schematisch dargestellt - einen Längsschnitt einer Darstellung einer Anordnung 201 für eine Elektrodenleitungs-Anschlussbuchse, welche eine Hülse 202 mit einer Hülsenwand 203, Feder-Kontaktelemente 230, 231, 232, 233, und einen Dichtungsring 211 mit einer Dichtungsringdicke 212 umfasst. Der Dichtungsring 211 weist einen kreisrunden Durchbruch 213 zur Aufnahme einer Elektrodenleitung mit rundem Querschnitt auf und ist derart geformt, dass die Hülsenwand gegen den Durchbruch 213 abgedichtet ist.

Die Hülse 202 weist eine in dem Hülseninnenraum angeordnete Trennwand 204 auf, welche den Hülseninnenraum entlang einer - in dieser Abbildung nicht dargestellten - Hülsenlängsachse teilt. Die Trennwand 204 weist einen zentrisch angeordneten kreisrunden Durchbruch zum Durchführen eines Elektrodenleitungs-Steckers auf. Der Durchmesser des Trennwand- Durchbruchs entspricht dem Durchmesser des Dichtungsscheibendurchbruchs 213.

Die Dichtungsscheibe 211 weist eine Dichtungsscheibendicke 212 auf, so dass der Durchbruch der Dichtungsscheibe 211 eine Durchbruch-Innenwand aufweist. Die Durchbruch-Innenwand weist zwei in Hülsenlängsachse hintereinander angeordnete Dichtlippen auf. Die Dichtlippen weisen jeweils radial nach innen und sind an die Dichtscheibe angeformt.

Figur 2b zeigt - schematisch dargestellt - eine Explosionsdarstellung der in Figur 2a in Längsschnitt dargestellten Anordnung für eine Elektrodenleitungsbuchse. Die Anordnung für eine Elektrodenleitungsbuchse umfasst eine Hülse 202, eine Dichtungsscheibe 211 und einen Feder-Kontaktelement-Träger 215 mit sechs angeformten Feder-Kontaktelementen 230, 231, 232, 233, 234 und 235.

Die Hülse 202 weist eine Hülsenwand 203 auf, welche einen Hülseninnenraum umfasst. Der Hülseninnenraum ist entlang der Hülsenlängsachse 214 zu den Hülsenenden wenigstens an einem Ende und im Ausführungsbeispiel an beiden Enden (Stirnseiten) geöffnet.

Die Hülse 202 weist eine Trennwand 204 auf, welche in dem Hülseninnenraum angeordnet und an die Hülsenwand 203 angeformt ist. Die Trennwand 204 weist einen zentrisch angeordneten kreisrunden Durchbruch zur Durchführung eines Elektrodenleitungssteckers entlang der Hülsenlängsachse 204 auf. Die Trennwand 204 ist von den Hülsenenden in Richtung der Hülsenlängsachse 204 jeweils beabstandet.

Der Feder-Kontaktelement-Träger 215 ist als kreisrunde flache Scheibe ausgebildet, welche einen zentrisch angeordneten Durchbruch zur Durchführung eines Elektrodenleitungssteckers aufweist.

Der Feder-Kontaktelement-Träger 215 weist sechs in Umfangsrichtung gleichmäßig angeordnete Feder-Kontaktelemente 230, 231, 232, 233, 234 und 235 auf, welche am äußeren Rand des Feder-Kontaktelement-Trägers 215 angeformt sind.

Die Feder-Kontaktelemente sind jeweils aus einer wellenförmig gewendeten Blattfeder gebildet, wobei die Blattfeder zwei Wendeabschnitte mit jeweils entgegengesetztem Wenderichtungssinn aufweist und so eine S-förmige Wellenform aufweist.

Die so ausgebildeten Feder-Kontaktelemente 230, 231, 232, 233, 234 und 235 sind ausgebildet, unter Auslenkung entlang der Federauslenkungsachse eine der Auslenkung entgegenwirkende Federkraft auszubilden und können - im Anwendungsfall - bei Einführung eines Elektrodenleitungssteckers federnd elastisch - im Wesentlichen radial - nach außen ausweichen.

Der Feder-Kontaktelement-Träger 215 weist in Umfangsrichtung jeweils seitlich an die Federkontaktelemente anschließende Aussparungs-Bereiche auf.

Der Feder-Kontaktelement-Träger weist auch in Umfangsrichtung an der Innenkante, welche an den Durchbruch des Feder-Kontaktelement-Trägers angrenzt, jeweils gegenüber eines jeden Federkontaktelements angeordnete Aussparungsbereiche auf.

In diesem Ausführungsbeispiel ist der Feder-Kontaktelement-Träger 215 und die Federkontaktelemente aus einem Blechstück gestanzt. Die Feder-Kontaktelemente sind jeweils aus strahlenförmig radial nach außen weisenden Blattfederstreifen gebildet, welche zur Ausbildung der Wellenform am Umfangsansatz des Feder-Kontaktelement-Trägers 215 rechtwinklig abgebogen und im weiteren Verlauf zu einer S-förmigen Wellenform ausgeformt sind.

Die Aussparungen seitlich der Federkontaktelemente sind derart tief in radiale Richtung eingeschnitten, dass das rechtwinklig abgebogene Federkontaktelement den äußeren Durchmesser des Feder-Kontaktelement-Trägers nicht überschreitet.

Die Aussparungen am inneren Rand des Feder-Kontaktelement-Trägers 215 zum Durchbruch hin sind derart tief in radiale Richtung nach außen eingeschnitten, dass ein noch stehender Bereich des Feder-Kontaktelement-Trägers 215 zwischen den äußeren und den inneren Aussparungen - gesehen in radialer Richtung - eine in tangentialer Richtung wirkende Torsionsfeder bildet.

Die so gebildete Torsionsfeder unterstützt die Federwirkung des Feder-Kontaktelements derart, dass die Torsionsfeder in Serie zu dem Feder-Kontaktelement wirkt. Die daraus resultierende Gesamtfedersteife, gebildet aus der Federsteife der Torsionsfeder und der Federsteife des Federkontaktelements, ist kleiner als die Federsteife des FederKontaktelements ohne die in Serie wirkende Torsionsfeder.

Der Feder-Kontaktelement-Träger 215 ist in seinem äußeren Umfangsdurchmesser derart bemessen, dass er entlang der Hülsenlängsachse in den Hülseninnenraum eingeführt werden kann.
Die Anordnung umfasst auch eine - in Figur 2a bereits beschriebene - Dichtungsscheibe 211, welche eine vom äußeren Dichtungsscheibenrand beabstandete Ring-Nut aufweist. Der Durchmesser der Ring-Nut entspricht dem Durchmesser der Hülse 202, sodass ein Ende der Hülsenwand 203 in Richtung der Hülsenlängsachse in die Ring-Nut der Dichtungsscheibe 212 eingeschoben werden kann.

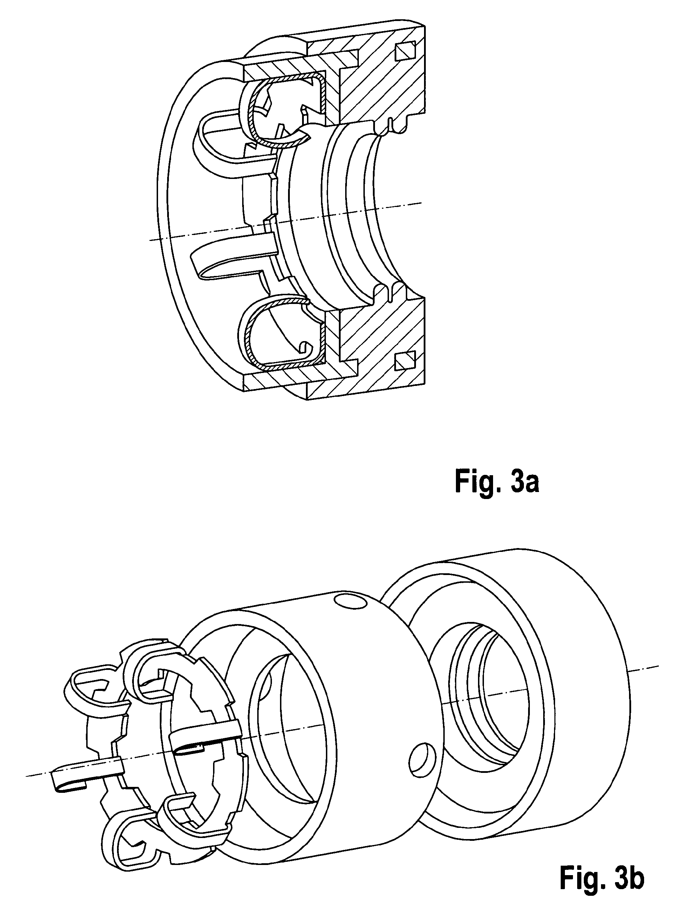

Die Ausführungsvariante gemäß Figuren 3a und 3b unterscheidet sich von der zuvor Beschriebenen durch die Gestaltung der Federkontaktelemente. Diese weisen jeweils nur einen Wendeabschnitt auf und sind C-förmig ausgebildet. Die vorteilhafte Kombination von einer Dichtscheibe mit einem Federkontaktelement-Träger mit nach innen ragenden Federkontaktelementen, die innerhalb einer gemeinsamen Hülse in Hülsenlängsrichtung hintereinander angeordnet sind, ist bei beiden Ausführungsvarianten gleichermaßen verwirklicht.

## Patentansprüche

1. Kontakt-Anordnung (101, 201) für eine Elektrodenleitungs-Anschlussbuchse eines implantierbaren Gerätes, umfassend eine Hülse (105),
mit einer Hülsenlängsachse (114, 214) und einer Hülsenwand (106, 203), wobei die Hülsenwand (106, 203) einen Hülseninnenraum (104) umschließt, welcher wenigstens eine Öffnung zum Einführen eines Elektrodenleitungs-Steckers entlang der Hülsenlängsachse (114, 214) aufweist,
**dadurch gekennzeichnet,**
**dass** die Hülse (105) eine Dichtung (211) aufweist, welche einen runden Durchbruch (213) zum Einführen eines Elektrodenleitungs-Steckers mit rundem Querschnitt aufweist und derart geformt ist, dass die Öffnung gegen die Hülsenwand (106, 203) und gegen den Durchbruch (213) abgedichtet ist.

2. Kontakt-Anordnung (101, 201) nach Anspruch 1, **dadurch gekennzeichnet, dass** die Dichtung (211) eine Dichtscheibe ist, und der Durchbruch (213) eine Durchbruchinnenwand aufweist, wobei die Durchbruchinnenwand wenigstens zwei in Richtung Hülsenlängsachse (114, 214) hintereinander angeordnete Dichtlippen aufweist, welche jeweils radial nach innen weisen und an die Dichtscheibe angeformt sind.

3. Feder-Kontaktelement-Anordnung für eine Kontakt-Anordnung nach Anspruch 1 oder 2, mit einem Feder-Kontaktelement-Träger (215), welcher mindestens drei angeformte Feder-Kontaktelemente (230, 231, 232, 233, 234, 235) aufweist, wobei der Feder-Kontaktelement-Träger (215) als kreisförmige flache Scheibe ausgebildet ist, welche einen Durchbruch zur Durchführung eines Steckers aufweist.

4. Feder-Kontaktelement-Anordnung nach Anspruch 3, **dadurch gekennzeichnet, dass** die Feder-Kontaktelemente (230, 231, 232, 233, 234, 235) am Feder-Kontaktelement-Träger (215) in Umfangsrichtung gleichmäßig angeordnet und im Bereich des äußeren Randes des Feder-Kontaktelement-Trägers (215) angeformt sind.

5. Feder-Kontaktelement-Anordnung nach Anspruch 3 oder 4, **dadurch gekennzeichnet, dass** die Feder-Kontaktelemente (230, 231, 232, 233, 234, 235) jeweils von einer wellenförmig gewendeten Blattfederzunge gebildet sind, wobei die Blattfederzunge zwei Wendeabschnitte mit jeweils einander entgegengesetztem Wenderichtungssinn aufweist und so eine S-förmige Wellenform hat und die Blattfederzungen ausgebildet sind, unter Auslenkung entlang der Federauslenkungsachse eine der Auslenkung entgegenwirkende Federkraft auszubilden und beim Einführen eines Steckers federnd elastisch nach außen auszuweichen.

6. Feder-Kontaktelement-Anordnung nach einem der Ansprüche 3 bis 5, **dadurch gekennzeichnet, dass** der Feder-Kontaktelement-Träger (215) in Umfangsrichtung jeweils seitlich an die Federkontaktelemente anschließende Aussparungs-Bereiche aufweist.

7. Feder-Kontaktelement-Anordnung nach einem der Ansprüche 3 bis 65, **dadurch gekennzeichnet, dass** der Feder-Kontaktelement-Träger (215) in Umfangsrichtung an der Innenkante, welche an den Durchbruch des Feder-Kontaktelement-Trägers angrenzt, jeweils gegenüber eines jeden Federkontaktelements angeordnete Aussparungsbereiche aufweist.

8. Feder-Kontaktelement-Anordnung nach einem der Ansprüche 3 bis 7, **dadurch gekennzeichnet, dass** der Feder-Kontaktelement-Träger (215) mit den Federkontaktelementen aus einem einstückigen Blech gebildet ist, welches strahlenförmig radial nach außen weisende Blattfederstreifen aufweist und die Feder-Kontaktelemente jeweils aus den strahlenförmig radial nach außen weisenden Blattfederstreifen gebildet sind, wobei die Blattfederstreifen-Ansätze am Umfang des Feder-Kontaktelement-Trägers (215) abgewinkelt und im weiteren Verlauf zu einer S-förmigen Wellenform ausgeformt sind.

9. Feder-Kontaktelement-Anordnung nach Anspruch 8, **dadurch gekennzeichnet, dass** die Blattfederstreifen am Umfangsansatz des Feder-Kontaktelement-Trägers (215) rechtwinklig abgewinkelt sind.

10. Feder-Kontaktelement-Anordnung nach einem der Ansprüche 8 oder 9, **dadurch gekennzeichnet, dass** die Aussparungen seitlich der Federkontaktelemente derart tief in radiale Richtung eingeschnitten sind, dass nach mindestens rechtwinkligem Abwinkeln eines Blattfederstreifens ein Abschnitt des Blattfederstreifens beginnend am Umfang des Feder-Kontaktelement-Trägers (215) einen äußeren Umfangsradius des Feder-Kontaktelement-Trägers (215) neben den Aussparungen nicht überschreitet.

11. Implantierbares Gerät mit einer Kontakt-Anordnung (101, 201) oder einer Feder-Kontaktelement-Anordnung nach einem der vorhergehenden Ansprüche, **dadurch gekennzeichnet, dass** das implantierbare Gerät ein Herzschrittmacher, ein Kardioverter, ein Defibrillator oder eine Kombination aus diesen ist.
